# EUROPEAN PATENT APPLICATION

(11) **EP 4 265 265 A1**
(43) Date of publication of application: **25.10.2023**
(21) Application number: 22382363.4
(22) Date of filing: 19.04.2022
(51) Int. Cl.: A61K 35/76, A61P 31/04, C12N 7/00

(54) **BACTERIOPHAGE SUITABLE FOR TREATING A BACTERIAL INFECTION CAUSED BY MYCOBACTERIUM ABSCESSUS**

(71) Applicant: Fundacion Instituto De Investigacion Sanitaria Fundacion Jimenez Diaz, 28040 Madrid (ES); Universidad Complutense De Madrid, 28040 Madrid (ES)
(72) Inventor: GARCÍA QUINTANILLA, Meritxell de Jesús, 28040 Madrid (ES); ESTEBAN MORENO, Jaime, 28040 Madrid (ES); GADEA GIRONÉS, Ignacio, 28040 Madrid (ES); FERNÁNDEZ ROBLAS, Ricardo, 28040 Madrid (ES); AGUILERA CORREA, John Jairo, 28040 Madrid (ES); BRONCANO LAVADO, Antonio, 28040 Madrid (ES)
(74) Representative: Hoffmann Eitle

(57) **Abstract**

**BACTERIOPHAGE SUITABLE FOR TREATING A BACTERIAL INFECTION CAUSED BY MYCOBACTERIUM ABSCESSUS.** The present invention refers to a bacteriophage deposited at the German Collection of Microorganisms and Cell Cultures GmbH (DSMZ) with deposit number DSM 34221, or a bacteriophage comprising a nucleic acid having at least 95% of identity with the nucleic acid of the bacteriophage DSM 34221. In a preferred embodiment, the bacteriophage is used for treating bacterial infections caused by *Mycobacterium abscessus.*

## Description

### FIELD OF THE INVENTION

The present invention refers to the medial field. Particularly, the present invention refers to a bacteriophage deposited at the German Collection of Microorganisms and Cell Cultures GmbH (DSMZ) with deposit number DSM 34221 on 17^{th} March 2022, or a bacteriophage comprising a nucleic acid having at least 95% of identity with the nucleic acid of the bacteriophage DSM 34221. In a preferred embodiment, the bacteriophage is used for treating bacterial infections caused by *Mycobacterium abscessus.*

### STATE OF THE ART

Importantly, *M. abscessus* is the most challenging species of mycobacteria due to its intrinsic and acquired bacterial resistance. In the last decade, *M. abscessus* infections have increased including pulmonary infections, systemic and/or disseminated infections, and skin and softtissue infections. In pulmonary infection, *M. abscessus* causes chronic respiratory diseases in patients suffering from underlying lung diseases, such as cystic fibrosis, bronchiectasis, and chronic obstructive pulmonary disease. This increment is due to a growing number of patients with immunosuppression and increased life expectancy, as well as other factors that predispose to these infections.

*Mycobacterium abscessus* presents a phenotypic heterogeneity based on the presence (smooth) or absence (rough) of glycopeptidolipids (GPLs) extracellularly, rough derivatives exhibiting higher levels of virulence than smooth variants.

Phage therapy is a promising afresh therapy, which uses viruses infecting bacteria to deal with infections. In 2014, the United States National Institute of Infectious Diseases included phage therapy as one of seven strategies to tackle antibiotic resistance.

Consequently, since *M. abscessus* is a challenging bacterium, there is an unmet clinical need of finding reliable strategies able to inhibit the growth of clinical strains of *M. abscessus* both in liquid and in mature biofilms. The present invention, which is precisely based on phage therapy, is focused on solving this problem by providing a new therapeutic strategy.

### DESCRIPTION OF THE INVENTION

### Brief description of the invention

The present invention refers to a bacteriophage deposited at the German Collection of Microorganisms and Cell Cultures GmbH (DSMZ) with deposit number DSM 34221 on 17^{th} March 2022, or a bacteriophage comprising a nucleic acid having at least 95% of identity with the nucleic acid of the bacteriophage DSM 34221, (hereinafter *"bacteriophage of the invention or F2MA").* In a preferred embodiment, the bacteriophage is used for treating bacterial infections caused by *M. abscessus.*

The bacteriophage of the invention was deposited at the German Collection of Microorganisms and Cell Cultures GmbH (DSMZ) on 17^{th} March 2022.

It is of utmost importance to consider that, according the results provided in the present invention (see **Example 2** of the present invention), the bacteriophage of the invention is able to inhibit the growth of clinical strains of *M*. *abscessus.* It is expected the bacteriophage of the invention is able to inhibit strains resistant to antibiotics both in liquid and in mature biofilms.

So, the first embodiment of the present invention refers to a bacteriophage deposited at the German Collection of Microorganisms and Cell Cultures GmbH (DSMZ) with deposit number DSM 34221, or a bacteriophage comprising a nucleic acid having at least 95% of identity with the nucleic acid of the bacteriophage DSM 34221.

The second embodiment of the present invention refers to the bacteriophage of the invention for use as a medicament, preferably for use in the prevention or treatment of a bacterial infection caused by *M. abscessus.*

The third embodiment of the invention refers to a pharmaceutical composition comprising the bacteriophage of the invention and, optionally, pharmaceutically acceptable excipients or carriers.

The fourth embodiment of the present invention refers to the pharmaceutical composition comprising the bacteriophage of the invention for use as a medicament, preferably for use in the prevention or treatment of a bacterial infection caused by *M. abscessus.*

Alternatively, the present invention refer to a method for preventing or treating a bacterial infection caused by *M. abscessus* which comprises the administration of a therapeutically effective dose or amount of the bacteriophage of the invention or a pharmaceutical composition comprising the bacteriophage of the invention.

In a preferred embodiment the bacteriophage of the invention is administered along with one or more antibiotics, wherein the antibiotics are administered before, after or the same time than the bacteriophage of the invention. The antibiotics to be administered in combination with the bacteriophage of the invention are preferably selected according to the antibiogram of the mycobacterium, generally two or three drugs are used whose sensitivity has been demonstrated in vitro as the options are very limited, but we can cite clarithromycin (although the resistance developed by the erm gene reduces the therapeutic options), amikacin, cefoxitin, imipenem, tigecycline and linezolid, or combinations thereof.

The last embodiment of the present invention refers to a method of detecting the presence of *M. abscessus* in an *in vitro* sample, for instance obtained from a subject, wherein the subject is preferably a patient, comprising contacting said sample with the bacteriophage of the invention and determining whether said bacteriophage kills bacteria in said sample.

For the purpose of the present invention the following terms are defined:
- The term "comprising" is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- By "therapeutically effective dose or amount" is intended an amount that, when administered as described herein, brings about a positive therapeutic response in the patient. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.

### Description of the figures

**Figure 1****.** pH stability of F2MA for 1 hat pH 1, 4.5, 7.4 and 8. The bars represent the median and the interquartile range. The horizontal dashed line represents the limit of detection.
**Figure 2****.** F2MA temperature stability. Incubation for 24 h and 168 h at temperatures of -80°C, -20°C, 4°C, 21°C, 37°C and 60°C. The bars represent the median and the interquartile range. The horizontal dashed line represents the limit of detection.
**Figure 3****.** Bacteriophage inhibition assays. Inhibition of one clinical isolate (330) after treatment with F2MA at a 500 multiplicity of infection (MOI).
**Figure 4****.** Bacteriophage inhibition assays. Inhibition of one clinical isolate (330) after treatment with F2MA at a 0.5 multiplicity of infection (MOI).
**Figure 5****.** Intracellular treatment of *M. abscessus* with phages. The bars represent the median and interquartile range. ^{∗}: p-value<0.05, ^{∗∗}: p-value<0.01. ^{∗∗∗}: p-value<0.001, ^{∗∗∗∗}: p-value<0.0001 for non-parametric Mann-Whitney test.

### Detailed description of the invention

The present invention is illustrated by means of the Examples set below without the intention of limiting its scope of protection.

### Example 1. Material and Methods

### Example 1.1. M. abscessus strains

Thirty clinical strains were studied from patients of the Fundación Jiménez Diaz Hospital isolated between 2007 - 2020. These strains were kept in refrigerated tanks at -80°C in skimmed milk stocks (East Rutherford, NJ, United States), and thawed on TSS blood agar (BioMerieux, France) at 37°C in CO₂ ovens for this study. Identification was performed by PCR assay with NTM hybridization, both at species and subspecies level. The sensitivity study was performed by microdilution according to CLSI recommendations.

### Example 1.2. Bacteriophage isolation

Phage against *M. abscessus* was isolated from waste waters of HUFJD (Madrid, Spain). Samples of these waters were centrifuged at 4500 g, then filtered with 0.22 µm filters to remove cellular debris. Then, an aliquot of 100 µl was incubated in 5 ml of TSB (Sigma-Adrich) together with the same volume of *M. abscessus* culture (previously incubated for 3 days in TSB) and 150 µl of calcium chloride (Thermo Fisher Scientific, Waltham, MA, USA) and magnesium sulphate (Thermo Fisher Scientific, Waltham, MA, USA) solution at 200 mM. This culture is then incubated for 3 days in a CO₂ oven at 37°C with agitation between 80 and 100 rpm. After this period of time, the culture is centrifuged at 4500 g for 20 minutes, the resulting supernatant is filtered through 0.40 µm and 0.22 µm filters and stored at 4°C. To culture the phages, they are seeded using the top agarose method by adding 3 ml of Top agarose TSB together with 150 µl of CaCl₂ and 200 mM MgSO₄ solution and 100 µl of liquid culture of *M. abscessus* in TSB, and 100 µl of the previous phytase. Repeat this process with 1:10 serial solutions of the filtrate. Finally, pour onto a TSS blood agar plate and incubate for three days. Any bald spots on the solid medium are cut with a Pasteur pipette, vortexed, centrifuged at 400 g in 2 ml of SM buffer 100 mM sodium chloride (Panreac Química, Illinois Tool Works, Glenview, Illinois United States) solution in an Eppendorf (Eppendorf, Hamburg, Germany), after which 800 µl are taken and preserved in glass.

### Example 1.3. Bacteriophage enrichment

Incubate 1 ml of liquid culture in TSB (three days of prior incubation) in 10 mL of liquid TSB medium along with 500 µl of CaCl₂ and 200 mM MgSO₄ solution and 500 µl of F2MA phage in a glass vial. They are incubated at 37° C at 80-100 rpm for three days. After this, they are centrifuged at 4500 rpm and filtered through 0.40 µm and 0.22 µm filters. Serial dilutions are made 1:10 and seeded by the Top agarose TSB method on TSS blood agar (Biomerieux) and incubated for 3 days to determine the resulting bacteriophage titer. The large-scale amplification was applied according to the state of the art.

### Example 1.4. Bacteriophage stability testing

The stability of F2MA was measured in SM solution at different pHs (1, 4.5, 7.4 and 8). For this purpose, a 10 µl aliquot was added to 1 mL of SM solution adjusted with HCl and 1M NaOH (Sigma-Aldrich, Castle Hill, NSW, Australia) to achieve the above pHs. The samples were incubated for one hour at room temperature. For the thermal stability study, 10 µl were added to 1 ml of TSB and the samples were exposed to the following temperatures: -80 °C, - 21 °C, 4 °C, 21 °C, 37 °C and 55 °C. The thermal stability testing was studied following the state of the art.

### Example 1.5. Bacteriophage Host Range analysis

The ability of F2MA to lyse *M. abscessus* clinical strains obtained from Hospital Fundación Jiménez Diaz, spot testing. Briefly, 3 µl of purified phage suspension (10⁹ pfu/ml) was spotted into the surface of double-layer agar plate previously inoculated with the tested clinical strains. After the adsorption of phage suspension, the plates were incubated 72 h at 7 °C. Host range was determined by plaque formation. Two replicates were performed with 30 clinical strains of *M. asbcessus.* The host range test was applied according to methodology previously described.

### Example 1.6. Bacteriophage inhibition assays

Growth curves of liquid culture of *M*. *abscessus* in the presence and absence of bacteriophages were performed. The variation of the absorbance of these cultures was measured in spectrophotometer at 570 nm every 24 h for five days.

### Example 1.7. Intracellular treatment of M. abscessus with phages

A liquid culture suspension of *M*. *abscessus* at 1:10 dilution is inoculated into a P48 plate with RAW 264.7 macrophages (1000 cells per well). After several washes with PBS, the bacteriophages are inoculated into alpha-MEM medium (with 10% fetal bovine serum and 1% penicillin and streptomycin) containing the bacteriophage. They are incubated 72 hours and washed with PBS solution and cells are lysed with injectable water. Bacterial cells are quantified by the Drop Plate method.

### Example 1.8. Statistical analysis

The distribution of the data was evaluated using the Shapiro-Wilk or Kolgomorov-Smirnov statistics. Descriptive statistics are quoted as median and interquartile range (non-normal distribution non-normal distribution) for each variable that was calculated. The Mann-Whitney nonparametric test considering the equality of variances was used to compare two groups and the nonparametric test Kruskal-Wallis test was used to compare more than two groups. To determine the effect of temperature on phage viability. Inhibition of bacterial biofilm by bacteriophages. All statistical analysis was performed using GraphPad Prism v.8 (GraphPad Prism, version 8.0.1 (86); Windows Version by Software MacKiev ^{©} 2020-2018 GraphPad Software, LLC.; San Diego, CA, USA) and STATA statistical software, release 11 (StataCorp, 2009, StataCorp LP., Texas, USA).

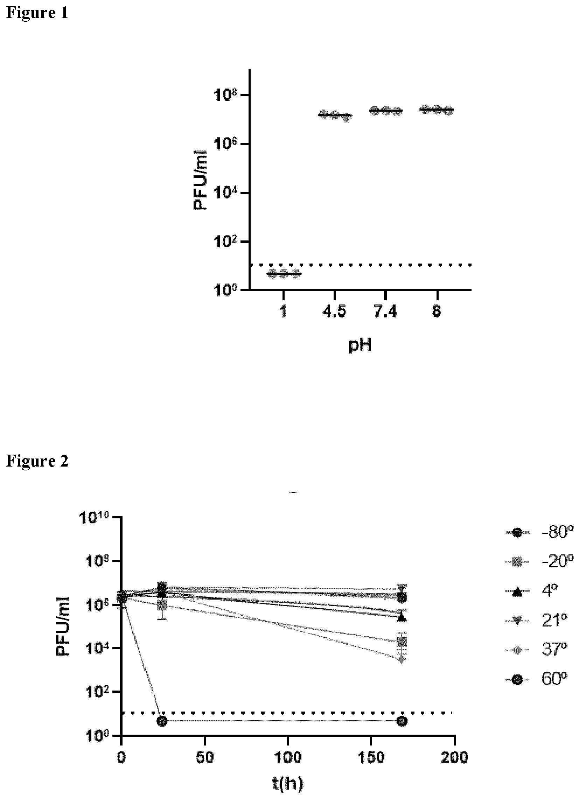

Departing from these materials and methods, and the common general knowledge, the present invention could be reproduced by the person skilled in the art. For instance, the reference [Jiang S, Tang Y, Xiang L, Zhu X, Cai Z, Li L, Chen Y, Chen P, Feng Y, Lin X, Li G, SharifJ, Dai J. Efficient de novo assembly and modification of large DNA fragments. Sci China Life Sci. 2021 Dec 16. doi: 10.1007/s11427-021-2029-0. Epub ahead of print. PMID: 34939159*]* explains how to synthesise de novo long DNA from sequences. On the other hand, the reference [Diana P. Pires et al., 2021. Designing P. aeruginosa synthetic phages with reduced genomes. Scientific Reports |(2021) 11:2164 | https://doi.org/10.1038/s41598-021-81580-2] explains how phages can be synthesized and a general scheme is provided therein (see Figure 2 of this paper).

### Example 2. Results

### Example 2.1. Bacteriophage isolation and bacteriophage Host Range analysis

The collection of 30 clinical isolates of *M. abscessus* contained 21 strains of *M. abscessus* subsp. *abscessus* (30%), 8 strains of *M. abscessus* subsp. *massiliense* (26%) and one strain of *M. abscessus* subsp. *bolletii* (4%). The mycobacteriophage was isolated. The bacteriophage infected 16 strains (53.3%) of the collection: *13 M. abscessus* subsp. *abscessus,* 2 *M. abscessus* subsp. *masilliense* and *one M. abscessus* subsp. *bolletii* as shown in **Table 1.**

**Table 1**

| **Strain** | **Subsp.** | **F2MA** | **Type** |
|---|---|---|---|
| **299** | *bolletii* | * | s |
| **330** | *abscessus* | * | R |
| **368** | *abscessus* | * | s |
| **376** | *abscessus* | * | s |
| **382** | *abscessus* | - | s |
| **383** | *abscessus* | * | R |
| **386** | *abscessus* | * | R |
| **408** | *abscessus* | * | s |
| **418** | *massiliense* | - | s |
| **420** | *abscessus* | * | R |
| **434** | *abscessus* | - | s |
| **442** | *abscessus* | * | R |
| **607** | *abscessus* | - | s |
| **611** | *massiliense* | - | s |
| **622** | *abscessus* | * | s |
| **623** | *massiliense* | - | s |
| **624** | *massiliense* | - | R |
| **636** | *abscessus* | - | s |
| **718** | *abscessus* | - | s |
| **726** | *abscessus* | - | R |
| **729** | *massiliense* | - | s |
| **757** | *massiliense* | - | s |
| **759** | *abscessus* | * | R |
| **776** | *abscessus* | * | s |
| **782** | *abscessus* | - | s |
| **783** | *abscessus* | * | R |
| **789** | *abscessus* | - | s |
| **793** | *abscessus* | * | R |
| **795** | *massiliense* | * | s |
| **807** | *massiliense* | * | s |

| | | | |
|---|---|---|---|
| *Summary of F2MA Host Range. Based on the degree of lysis on the bacterial lawn, the spots were differentiated into two categories: susceptible (*^{∗}*) or non-susceptible (-).S: Smooth; R: Rough* | | | |

### Example 2.2. Bacteriophage stability testing

The phage F2MA was stable at most of the pH at which they were exposed for one hour with the exception of pH 1 at which no indication of bacteriophage growth was obtained according to the statistical model employed (Fig. 1). Regarding thermal stability, the isolated phage was stable at most of the temperatures tested after 24 h and 168 h of exposure, however, at -20°C and 37°C significant differences were observed since at these temperatures they were found to be unstable: F2MA degraded 0.41% and 0.43% respectively at temperatures of -20 °C and 37 °C, respectively **(****Fig. 2****).** In turn, the bacteriophage was also unstable at 60 °C.

### Example 2.3. Bacteriophage inhibition assays

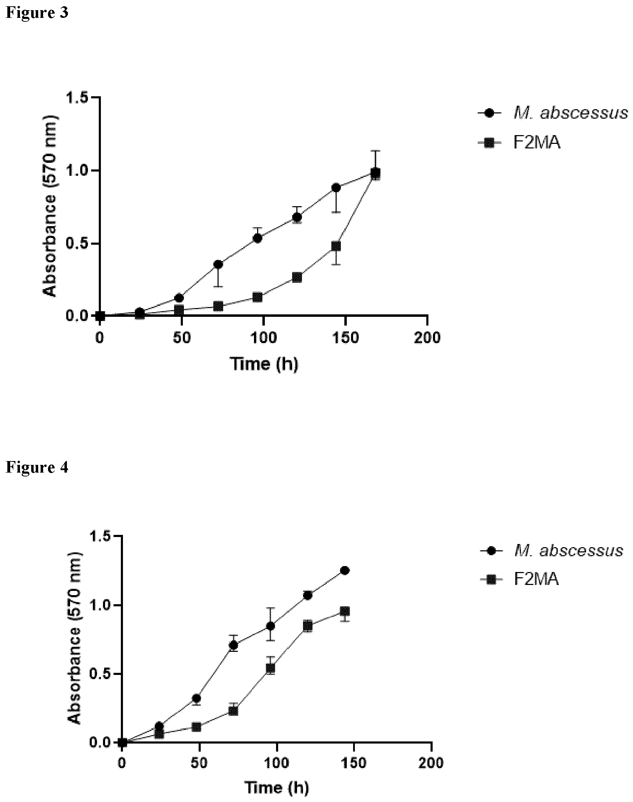

F2MA produces an inhibitory effect in growth of *M. abscessus.* A reduction in mycobacterial growth is observed both at a multiplicity of infection (MOI) of 500 **(****Fig. 3****)** and at an MOI of 0.5 **(****Fig. 4****),** although the reduction in bacterial growth is greater over time in the first case **(****Fig. 3****),** although in both cases it can be observed that F2MA becomes less effective over time, probably due to the selection of mycobacteria acquiring resistance to the bacteriophage.

### Example 2.4. Intracellular treatment of M. abscessus with phages in macrophages

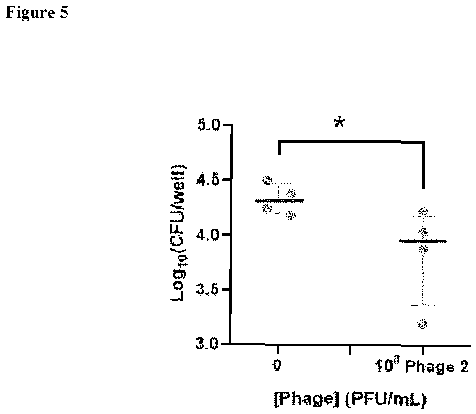

After a 24-hour treatment, F2MA was able to reduce the mycobacterial concentration inside the fibroblast significantly with respect to the control group using a titer of 10⁸ pfu/ml **(****Fig. 5****).** This suggests that F2MA can be internalized inside the eukaryotic macrophages where the mycobacterium is located and can target the bacteria intracellularly.

## Claims

1. Bacteriophage deposited at the German Collection of Microorganisms and Cell Cultures GmbH (DSMZ) with deposit number DSM 34221, or a bacteriophage comprising a nucleic acid having at least 95% of identity with the nucleic acid of the bacteriophage DSM 34221.

2. Bacteriophage, according to claim 1, with deposit number at the German Collection of Microorganisms and Cell Cultures GmbH (DSMZ) DSM 34221.

3. Bacteriophage, according to any of the previous claims, for use as a medicament.

4. Bacteriophage for use, according to claim 3, in the prevention or treatment of a bacterial infection caused by *M. abscessus.*

5. Pharmaceutical composition comprising the bacteriophage of any of the claims 1 or 2 and, optionally, pharmaceutically acceptable excipients or carriers.

6. Pharmaceutical composition of claim 5 for use as a medicament.

7. Pharmaceutical composition for use, according to claim 6, in the prevention or treatment of a bacterial infection caused by *M. abscessus.*

8. A method of detecting the presence of *M. **abscessus*** in an *in vitro* sample, comprising contacting said sample with the bacteriophages according to any of the claims 1 or 2, and determining whether said bacteriophages kill bacteria in said sample.
